# EUROPEAN PATENT APPLICATION

(11) **EP 4 629 591 A1**
(43) Date of publication of application: **08.10.2025**
(21) Application number: 23914796.0
(22) Date of filing: 26.12.2023
(51) Int. Cl.: H04L 51/06, G06F 3/01

(54) **INFORMATION TRANSMISSION DEVICE, INFORMATION TRANSMISSION METHOD, AND PROGRAM**

(30) Priority: 04.01.2023 JP 2023000026
(71) Applicant: JVCKenwood Corporation, Yokohama-shi, Kanagawa 2210022 (JP)
(72) Inventor: SHIMAKURA, Takamitsu, Yokohama-shi, Kanagawa 221-0022 (JP)
(74) Representative: Klang, Alexander H.
(86) International application number: PCT/JP2023/046531
(87) International publication number: WO 2024/147310

(57) **Abstract**

An information transmission apparatus includes a first information acquisition unit (31) configured to acquire first brain activity information of a first user; a second information acquisition unit (31) configured to acquire second brain activity information of a second user; a determination unit (33) configured to determine whether a difference between the first brain activity information and the second brain activity information is equal to or larger than a preset first determination value; and an information controller (34) configured to change a capacity of content transmitted and received between the first user and the second user based on a result of the determination by the determination unit.

## Description

### Field

The present disclosure relates to an information transmission apparatus, an information transmission method, and a program.

### Background

In recent years, a technique for measuring brain information has developed, and a brain-machine-interface (BMI) that is an interface directly connecting a brain and a machine becomes realistic. As such a technique, for example, there is a technology described in Patent Literature 1. Patent Literature 1 discloses a system which provides a command signal for electroencephalogram-based communication based on steady-state visual evoked response potential signals which are generated when a user is stimulated and which are acquired by multiple bioelectric sensors.

### Citation List

### Patent Literature

Patent Literature 1: Japanese Patent Application Laid-open No. 2013-117957 A

### Summary

Incidentally, there is a difference in understanding ability by the stimulated brain to understand things among individuals. In other words, there are a so-called person who is quick thinking and a so-called person who is slow thinking. Therefore, for conversation by using brain information, it is desired to adjust an information amount according to the person who is quick thinking or the person who is slow thinking.

The present disclosure solves the above problem, and an object of the present disclosure is to provide an information transmission apparatus, an information transmission method, and a program that facilitate conversation using brain information between multiple people.

According to one aspect of the present disclosure, there is provided an information transmission apparatus comprising: a first information acquisition unit configured to acquire first brain activity information of a first user; a second information acquisition unit configured to acquire second brain activity information of a second user; a determination unit configured to determine whether a difference between the first brain activity information and the second brain activity information is equal to or larger than a preset first determination value; and an information controller configured to change a capacity of content transmitted and received between the first user and the second user based on a result of the determination by the determination unit.

According to one aspect of the present disclosure, there is provided an information transmission method comprising: acquiring first brain activity information of a first user; acquiring second brain activity information of a second user; determining whether a difference between the first brain activity information and the second brain activity information is equal to or larger than a preset first determination value; and changing a capacity of content transmitted and received between the first user and the second user based on a result of the determination.

According to one aspect of the present disclosure, there is provided a program for causing a computer to execute: acquiring first brain activity information of a first user; acquiring second brain activity information of a second user; determining whether a difference between the first brain activity information and the second brain activity information is equal to or larger than a preset first determination value; and changing a capacity of content transmitted and received between the first user and the second user based on a result of the determination.

According to the information transmission apparatus, the information transmission method, and the program of the present disclosure, it is possible to facilitate conversation using the brain information between the multiple people.

### Brief Description of Drawings

FIG. 1 is a block configuration diagram illustrating an information transmission apparatus according to a first embodiment;
FIG. 2 is a block configuration diagram illustrating an information processing device;
FIG. 3 is a block configuration diagram illustrating an information controller;
FIG. 4 is an explanatory diagram illustrating an information multiplexing process;
FIG. 5 is a flowchart illustrating a reproduction control method;
FIG. 6 is a flowchart illustrating a multiplexing determination method;
FIG. 7 is a flowchart illustrating a multiplexing method in an information transmission apparatus according to a second embodiment; and
FIG. 8 is a flowchart illustrating a multiplexing method in an information transmission apparatus according to a third embodiment.

### Description of Embodiments

Hereinafter, preferred embodiments of the present disclosure will be described in detail with reference to the drawings. Note that the present disclosure is not limited to the embodiments, and the present disclosure also includes a configuration in which multiple embodiments is combined with each other. Furthermore, the following embodiments include component elements that are readily conceivable by a person skilled in the art, component elements that are substantially the same, and component elements that are within a so-called equivalent range.

### [First Embodiment]

### <Information transmission apparatus>

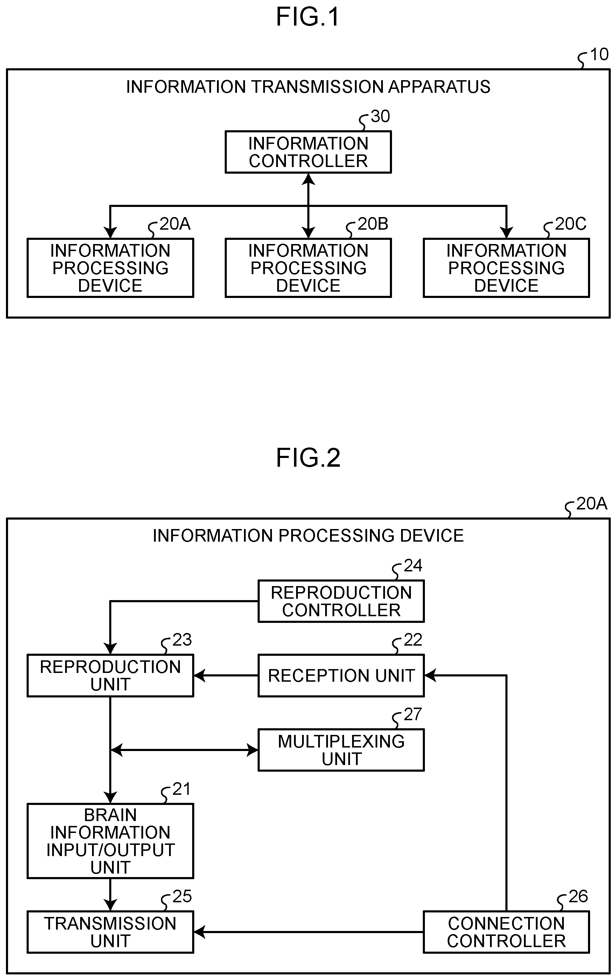

FIG. 1 is a block configuration diagram illustrating an information transmission apparatus according to a first embodiment.

As illustrated in FIG. 1, the information transmission apparatus 10 includes multiple (three in the first embodiment) information processing devices 20A, 20B, and 20C, and an information controller 30. The information processing devices 20A, 20B, and 20C are worn by a first user, a second user, and a third user. The information controller 30 is, for example, a server device. The information processing devices 20A, 20B, and 20C and the information controller 30 are connected via, for example, a wireless communication network. In the first embodiment, the first user (the information processing device 20A), the second user (the information processing device 20B), and the third user (the information processing device 20C) transmit information via the information controller 30. Note that, here, the number of users (information processing devices) is three, but may be two or four or more.

### <Information processing device>

FIG. 2 is a block configuration diagram illustrating an information processing device. Hereinafter, the information processing devices 20A, 20B, and 20C will be described, but the information processing devices 20A, 20B, and 20C have similar configurations, and therefore, the information processing device 20A will be described as a representative.

As illustrated in FIG. 2, the information processing device 20A includes a brain information input/output unit 21, a reception unit 22, a reproduction unit 23, a reproduction controller 24, a transmission unit 25, and a connection controller 26.

The brain information input/output unit 21 encodes information (reception command) into an electroencephalogram of a user and inputs the information to brain electrodes, and detects a transmission command based on the information decoded from the electroencephalogram acquired from the brain electrodes. At this time, the brain information input/output unit 21 adds a sender identification signal to the detected transmission command to form a message. The brain information input/output unit 21 is, for example, BMI.

The brain information input/output unit 21 acquires, for example, the electroencephalogram that is brain information of each user. The brain information input/output unit 21 includes, for example, electric sensors (e.g., electrodes) that detects an electroencephalogram emitted by a weak current flowing through a neural network of the brain. The brain information input/output unit 21 detects a potential (an electric signal) of the weak current generated when the user receives a stimulus from the outside or based on user's thinking such as a thought. Note that the brain information input/output unit 21 is not limited to detect the electroencephalogram. For example, the brain information input/output unit 21 may acquire an amount of blood flow caused by, for example, brain activity which is the brain information of the user, for example, by near infrared measurement or the like. Furthermore, the brain information input/output unit 21 may acquire histamine H3 receptor density in the frontal cortex of the user.

The brain information input/output unit 21 includes an electroencephalogram decoder. The electroencephalogram decoder restores the electric signal of the acquired electroencephalogram of the user to the information about user's thinking. In this case, a relationship between multiple electric signals of the electroencephalogram of the user and the information on user's thinking is associated in advance. For example, it is preferable to use machine learning using deep learning or the like to associate the relationship between the electric signals of the electroencephalogram and the information on user's thinking.

The brain information input/output unit 21 inputs, for example, the electroencephalogram as the brain information, to the user. The electroencephalogram decoder converts received information on user's thinking into the electric signal of the electroencephalogram of the user. The brain information input/output unit 21 encodes the electric signal of the electroencephalogram into the electroencephalogram and inputs the electroencephalogram to the brain electrode. Furthermore, the brain information input/output unit 21 may include a transcranial magnetic stimulator. In this case, the electroencephalogram decoder of the brain information input/output unit 21 encodes the electric signal of the electroencephalogram into the electroencephalogram to input into the brain magnetically.

The reception unit 22 receives a message from the outside, specifically, the information controller 30 and accumulates the messages. The reproduction unit 23 is connected to the reception unit 22.

The reproduction unit 23 removes receiver identification information from the message received by the reception unit 22. The reproduction unit 23 is connected to the brain information input/output unit 21. The reproduction unit 23 inputs the message from which the receiver identification information has been removed, to the brain information input/output unit 21, by a reproduction speed which is the same as that of the information decoded from the brain information input/output unit 21.

When reproducing the message, if the message is transmitted from a sender different from a sender of a previous message, the reproduction unit 23 records the current electroencephalogram of the user, causes the brain information input/output unit 21 to output the electroencephalogram of the user stored upon communication with the sender of the previous message, and then starts to reproduce the message.

The reproduction controller 24 is connected to the reproduction unit 23. The reproduction controller 24 controls a timing of inputting the message processed by the reproduction unit to the brain information input/output unit 21. Details of a process of the reproduction controller 24 will be described later.

The transmission unit 25 is connected to the brain information input/output unit 21. When the message is input from the brain information input/output unit 21, the transmission unit 25 transmits the message to the outside, specifically, the information controller 30. The transmission unit 25 is, for example, a communication module that transmits information in a wired or wireless manner.

The connection controller 26 is connected to the reception unit 22 and the transmission unit 25. The connection controller 26 controls connection of the reception unit 22 and the transmission unit 25 to the outside, specifically, the information controller 30.

Note that each of the reproduction unit 23, the reproduction controller 24, and the connection controller 26 includes a processor such as a central processing unit (CPU), and a storage such as a random access memory (RAM) or a read only memory (ROM). The reproduction unit 23, the reproduction controller 24, and the connection controller 26 may be integrated with each other or may be separated from each other. Alternatively, the reproduction unit 23 may include a memory or the like to record the current electroencephalogram of the user.

The message received by the reception unit 22 and the message transmitted by the transmission unit 25 are obtained by converting the electroencephalogram into a standard form and adding header information. The header information includes the sender identification signal, a receiver identification signal, control information, and the like. The control information is information indicating meaning of the electroencephalogram, such as "I think like this" or **"I** ask you for this".

### <Information controller>

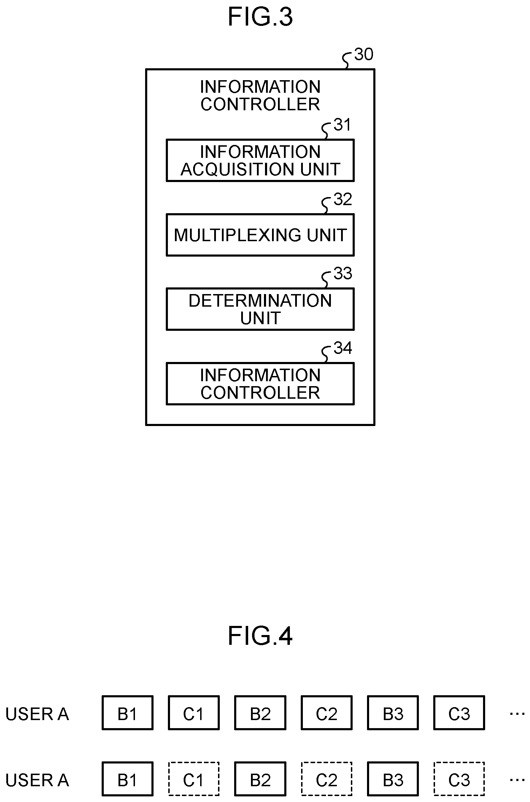

FIG. 3 is a block configuration diagram illustrating the information controller.

As illustrated in FIG. 3, the information controller 30 includes an information acquisition unit 31, a multiplexing unit 32, a determination unit 33, and an information controller 34.

The information acquisition unit 31 is connected to the information processing devices 20A, 20B, and 20C. The information acquisition unit 31 acquires multiple pieces of brain activity information of the users from the information processing devices 20A, 20B, and 20C. The information acquisition unit 31 acquires the electroencephalogram, the amount of blood flow caused by the brain activity, and the histamine H3 receptor density in the frontal cortex that are acquired by the brain information input/output unit 21 (see FIG. 2), in each of the information processing devices 20A, 20B, and 20C. The information acquisition unit 31 is, for example, a communication module that transmits information in a wired or wireless manner.

The multiplexing unit 32 multiplexes pieces of information (content) transmitted from the users. The multiplexing unit 32 of the first embodiment performs a time division multiplexing processing on the information. In other words, the multiplexing unit 32 divides information into multiple pieces, and transmits the divided pieces of the divided information with being shifted in time. A process of the multiplexing unit 32 will be described later.

The determination unit 33 calculates, for example, a difference between first brain activity information acquired from the information processing device 20A and second brain activity information acquired from the information processing device 20B. The determination unit 33 determines whether the calculated difference is equal to or larger than a preset first determination value. For example, the determination unit 33 determines whether a difference between a first histamine H3 receptor density as the first brain activity information and a first histamine H3 receptor density as the second brain activity information is equal to or larger than the preset first determination value.

The histamine H3 receptor in human frontal cortex is related to working memory, and the lower histamine H3 receptor density in the frontal cortex the human has, the higher frontal cortex activity important to the working memory is. In other words, the higher the histamine H3 receptor density in the frontal cortex is, the quicker thinking the person is. The determination unit 33 compares multiple pieces of the brain activity information to calculate, as a difference, an amount of difference in understanding ability (understanding speed) among multiple users. The first determination value is set in advance, and it is preferably set by acquiring the brain activity information of a large number of users and using an average, standard deviation, or the like thereof.

The information controller 34 changes an information (content) capacity transmitted and received among multiple users based on a result of determination by the determination unit 33. In other words, upon transmission and reception of the information among users who have equivalent minds or from a user who is slow thinking to a user who is quick thinking, the information controller 34 does not change the information (content) capacity transmitted and received among the multiple users. In contrast, upon transmission and reception of information from the user who is quick thinking to the user who is slow thinking, the information (content) capacity transmitted and received among the multiple users is reduced.

For example, when it is determined that the difference is equal to or larger than the first determination value, the information controller 34 summarizes information transmitted from the user who is quick thinking to the user who is slow thinking. Various techniques are preferably used for the summarization. Note that multiple determination values may be provided so that the number of characters is reduced as the difference increases. Furthermore, for example, when it is determined that the difference is equal to or larger than the first determination value, the information controller 34 reduces the reproduction speed of the information transmitted from the user who is quick thinking to the user who is slow thinking. Various techniques are preferably used for reducing the reproduction speed. Note that multiple determination values may be provided so that the reproduction speed is increased as the difference increases. Then, when it is determined that the difference is equal to or larger than the first determination value, the information controller 34 transmits the multiplexed information (content). Therefore, it is possible to simultaneously establish a conversation among the users who are quick thinking (the users for whom the reproduction speed is relatively fast) and the users who are slow thinking (the users for whom the reproduction speed is relatively slow), and a call in the time division multiplexing processing can be performed.

Note that each of the multiplexing unit 32, the determination unit 33, and the information controller 34 includes a processor such as a central processing unit (CPU), and a storage such as a random access memory (RAM) or a read only memory (ROM). The reproduction unit 23, the reproduction controller 24, and the connection controller 26 may be integrated with each other or may be separated from each other.

### <Multiplexing process>

FIG. 4 is an explanatory diagram illustrating an information multiplexing process.

As illustrated in FIG. 4, when a user A, a user B, and a user C have a conversation by using the information transmission apparatus 10, the user A receives the information of the user B and the information of the user C. At this time, the time division multiplexing processing is performed, and the user A receives information B1 of the user B, information C1 of the user C, information B2 of the user B, information C2 of the user C, information B3 of the user B, and information C3 of the user **C...,** in this order.

At this time, when a difference between brain activity information of the user A and brain activity information of the user B, and a difference between the brain activity information of the user A and brain activity information of the user C ((brain activity information of the user A) - (brain activity information of the user B), and (brain activity information of the user A) - (brain activity information of the user C)) are smaller than the first determination value, the information B1 of the user B, the information C1 of the user C, the information B2 of the user B, the information C2 of the user C, the information B3 of the user B, and the information C3 of the user C... are transmitted to the user A in this order. In contrast, when the difference between the brain activity information of the user A and the brain activity information of the user **B,** and the difference between the brain activity information of the user A and the brain activity information of the user C are equal to or larger than the first determination value, for example, the information B1 of the user **B,** the information B2 of the user **B,** and the information B3 of the user B are transmitted to the user A in this order, or the information C1 of the user **C,** the information C2 of the user **C,** and the information C3 of the user **C...** are transmitted to the user A in this order.

Note that the first determination value is used to be compared with the difference between the brain activity information of the user A and the brain activity information of the user **B,** and the difference between the brain activity information of the user A and the brain activity information of the user **C,** but the determination value is not limited to the first determination value. The difference between the brain activity information of the user A and the brain activity information of one of the user B or the user C may be compared to determine whether the difference is smaller than the first determination value or equal to or larger than the first determination value. Alternatively, the difference between the brain activity information of the user A and an average value of the brain activity information of the user B and the user C may be compared to determine whether the difference is smaller than the first determination value or equal to or larger than the first determination value.

### <Reproduction control method>

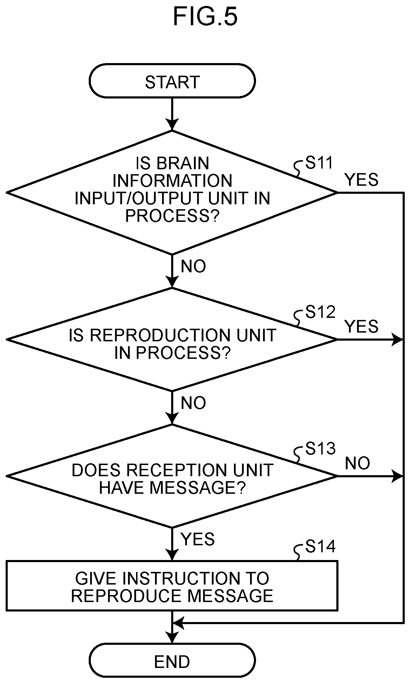

FIG. 5 is a flowchart illustrating a reproduction control method.

As illustrated in FIGS. 2 and **5****,** in Step S11, the reproduction controller 24 determines whether the brain information input/output unit 21 is in process, that is, whether the transmission command is being output or the reception command is being input. When it is determined that the brain information input/output unit 21 is in process (Yes), the reproduction controller 24 exits this routine. In contrast, when it is determined that the brain information input/output unit 21 is not in process (No), the reproduction controller 24 proceeds to Step S12.

In Step S12, the reproduction controller 24 determines whether the reproduction unit 23 is in process. When it is determined that the reproduction unit 23 is in process (Yes), the reproduction controller 24 exits this routine. In contrast, when it is determined that the reproduction unit 23 is not in process (No), the reproduction controller 24 proceeds to Step S13.

In Step S13, the reproduction controller 24 determines whether the reception unit 22 has a message. When it is determined (No) that the reception unit 22 has no message, the reproduction controller 24 exits this routine. In contrast, when it is determined that the reception unit 22 has the message (Yes), the reproduction controller 24 proceeds to Step S14. Then, in Step S14, the reproduction controller 24 controls the reproduction unit 23 by instructing the reproduction unit 23 to reproduce the messages in order from the oldest message among the messages stored in the reception unit 22.

At this time, when receiving a message from a sender to whom a connection is not established, the connection controller 26 adds the message to the reception unit 22 and establishes the connection when information transmitted to and received from the brain information input/output unit 21 is equal to or smaller than a predetermined rate, and otherwise, transmits a message indicating that processing is being performed to the outside.

Furthermore, when the brain information input/output unit 21 transmits a message requesting disconnection, the connection controller 26 disconnects the connection with a receiver of the message. Furthermore, when a message requesting disconnection is received from the outside, the connection controller 26 disconnects the connection with a sender of the message. Therefore, it is possible to simultaneously establish connection among the users for whom the reproduction speed is relatively fast and the users for whom the reproduction speed is relatively slow, and a call in the time division multiplexing processing can be performed.

Note that in the above description, a scheduling algorithm used by the reproduction controller 24 is a first-come, first-served method, but another method may be used.

### <Multiplexing determination method>

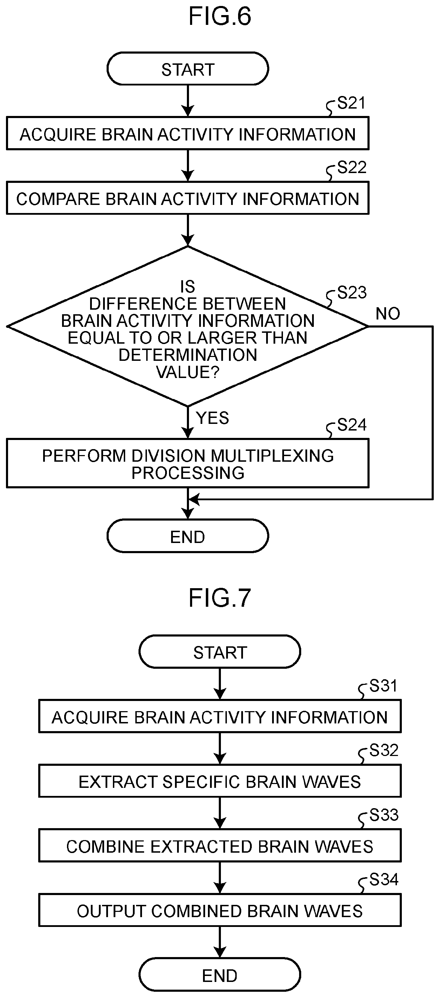

FIG. 6 is a flowchart illustrating a multiplexing determination method.

As illustrated in FIGS. 3 and **6****,** in Step S21, the information acquisition unit 31 acquires multiple pieces of brain activity information of the users from the information processing devices 20A, 20B, and 20C. In Step S22, the determination unit 33 compares the acquired multiple pieces of brain activity information to calculate differences. In Step S23, the determination unit 33 determines whether each of the calculated differences is equal to or larger than the first determination value. When it is determined that the difference is not equal to or larger than the first determination value (No), the determination unit 33 exits this routine. In other words, the multiplexing unit 32 does not perform the division multiplexing processing on the information (content), and the information controller 34 does not change the information (content) capacity.

In contrast, when it is determined that the difference is equal to or larger than the first determination value (Yes), the determination unit 33 proceeds to Step S24. In Step S24, the multiplexing unit 32 performs the division multiplexing processing on the information (content), and the information controller 34 reduces the information (content) capacity.

### [Second Embodiment]

FIG. 7 is a flowchart illustrating a multiplexing method in the information transmission apparatus according to a second embodiment. Note that the basic configuration of the second embodiment is similar to that of the first embodiment described above and will be described with reference to FIG. 3. In addition, members having similar functions to those of the first embodiment described above are denoted by the same reference numerals, and details thereof will be omitted.

As illustrated in FIG. 3, the information controller 30 includes an information acquisition unit 31, a multiplexing unit 32, a determination unit 33, and an information controller 34. In the second embodiment, the multiplexing unit 32 performs a frequency division multiplexing processing.

In other words, when a conversation is made among the first user (the information processing device 20A), the second user (the information processing device 20B), and the third user (the information processing device 20C), the information controller 30 allocates a frequency for each predetermined data unit to perform simultaneous transmission. In the second embodiment, element information being frequency division multiplexed is, for example, includes a α wave, a β wave, and a θ wave that are related to sensory information.

As illustrated in FIGS. 3 and 7, in Step S31, the information acquisition unit 31 acquires the electroencephalogram as the brain activation information from each of the information processing devices 20A, 20B, and 20C. In Step S32, the multiplexing unit 32 extracts specific brain waves having different frequencies from the acquired electroencephalograms of the users. In this case, the specific brain waves having different frequencies are, for example, the α wave, the β wave, and the θ wave, which are related to the sensory information. The multiplexing unit 32 combines the extracted multiple brain waves, and outputs the combined brain waves. For example, when the user A, the user B, and the user C have a conversation by using the information transmission apparatus 10, the user A receives the information of the user B and the information of the user C. At this time, the multiplexing unit 32 extracts the α wave from the electroencephalogram of the user B and the θ wave from the electroencephalogram of the user C, and outputs the α wave of the user B and the θ wave of the user C that have been combined. At this time, the multiplexing unit 32 preferably combines the multiple brain waves extracted from the multiple users by weighted averaging at a predetermined ratio. Therefore, the user A is allowed to simultaneously share the information of the users B and C.

Note that the processing of the determination unit 33 and the information controller 34 is similar to that of the first embodiment.

### [Third Embodiment]

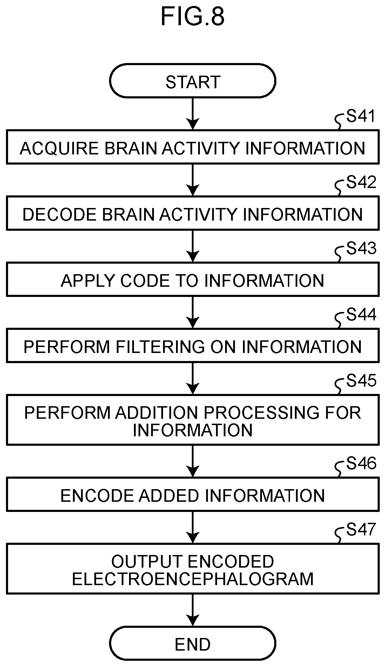

FIG. 8 is a flowchart illustrating a multiplexing method in an information transmission apparatus according to the second embodiment. Note that the basic configuration of the second embodiment is similar to that of the first embodiment described above, which will be described with reference to FIGS. 1 to 3, members having functions similar to those of the first embodiment described above are denoted by the same reference numerals, and detailed description thereof will be omitted.

As illustrated in FIG. 3, the information controller 30 includes an information acquisition unit 31, a multiplexing unit 32, a determination unit 33, and an information controller 34. In a third embodiment, the multiplexing unit 32 performs a code division multiplexing processing.

In other words, when a conversation is made among the first user (the information processing device 20A), the second user (the information processing device 20B), and the third user (the information processing device 20C), the information controller 30 applies an identifiable code to information for each predetermined data unit for simultaneous multiplexing transmission by a preset unit length. Here, the identifiable code has a unique pattern that does not occur in an encoded signal.

As illustrated in FIGS. 3 and 8, in Step S41, the information acquisition unit 31 acquires the electroencephalogram as the brain activation information from each of the information processing devices 20A, 20B, and 20C. In Step S42, the information acquisition unit 31 decodes the brain activation information. In Step S43, the multiplexing unit 32 applies codes to specific different pieces of information extracted from the acquired electroencephalograms of the users. In Step S44, filtering is performed on the different piece of information of the users to extract only the pieces of information to which the codes are applied.

In Step S45, the multiplexing unit 32 performs addition processing for the multiple pieces of information of the users to which the codes are applied. Here, for example, voice information and video information are combined. At this time, the multiplexing unit 32 specifies filter to pass elements having different pieces of information. In other words, pieces of information having an exclusive relationship, such as different topics, are specified, based on the filtering in Step S44. For example, when voice and video are specified, information obtained by decoding a signal of the auditory cortex is transmitted from the user A, and information obtained by decoding a signal of the visual cortex is transmitted from the user B. In Step S46, information obtained after adding is encoded, and in Step S47, the encoded electroencephalogram is output. Therefore, the user A is allowed to simultaneously share the information of the users B and C.

Note that, in the filtering in Step S44, a configuration in which topics that can or cannot be transmitted or received, or filters with which the information can or cannot be transmitted or received are set in a gateway for each user may be employed.

Note that the processing of the determination unit 33 and the information controller 34 is similar to that of the first embodiment.

Note that, in Step S23 of FIG. 6, the determination unit 33 determines whether the calculated difference is equal to or larger than the first determination value, and exits this routine when it is determined that the difference is not equal to or larger than the first determination value, but the determination unit 33 is not limited to this configuration. When it is determined that the difference is equal to or smaller a second determination value, the determination unit 33 may exit this routine, and further, the information controller 34 may generate and transmit a signal to the information processing device 20A, the information processing device 20B, to stop transmission of information, that is a message, to the outside, specifically to the information controller 30. Therefore, when the user A receives the information of the user B and the information of the user C, where the brain activity information of the user A is small and the brain activity information of the user B and the brain activity information of the user C are large, it is possible to prevent transmission of the messages of the user B and the user C to the user A and prevent the user A from being overloaded.

### [Operation and effects of present embodiment]

The information transmission apparatus of the present embodiment includes a first information acquisition unit 31 configured to acquire first brain activity information of a first user and second brain activity information of a second user, a determination unit 33 configured to determine whether a difference between the first brain activity information and the second brain activity information is equal to or larger than a preset first determination value, and an information controller 34 configured to change a capacity of content transmitted and received between the first user and the second user based on a result of the determination by the determination unit 33.

The information transmission apparatus of the present embodiment changes the capacity of the content based on the difference among the multiple pieces of brain activity information of the users, and therefore, for example, when information is transmitted among persons who have different minds, an appropriate capacity of the content is transmitted, and it makes possible to facilitate conversation among the multiple users by using the brain information.

In the information transmission apparatus of the present embodiment, when it is determined that the difference is equal to or larger than the first determination value, the information controller 34 summarizes the content. This configuration enables transmission of the appropriate amount of content among persons who have different minds.

In the information transmission apparatus of the present embodiment, when it is determined that the difference is equal to or larger than the first determination value, the information controller 34 changes the reproduction speed of the content. This configuration enables transmission of the content at an appropriate speed among persons who have different minds.

The information transmission apparatus of the present embodiment includes the multiplexing unit 32 that multiplexes content, and when it is determined that the difference is equal to or larger than the first determination value, the information controller 34 transmits the multiplexed content. This configuration enables appropriate transmission of the content among a large number of users.

Note that, in the embodiments described above, the information transmission apparatus 10 includes the multiple information processing devices 20A, 20B, and 20C, and the information controller 30, the information processing devices 20A, 20B, and 20C are worn by the users, and the information controller 30 serves as the server device. However, the present invention is not limited to this configuration. Instead of using the information controller 30 as the server device, each of the information processing devices 20A, 20B, and 20C may have the function of the information controller 30.

### Industrial Applicability

The information transmission apparatus, the information transmission method, and the program of the present disclosure are applicable to a technique for controlling a user's brain activity.

### Reference Signs List

- 10: INFORMATION TRANSMISSION APPARATUS
- 20A, 20B, 20C: INFORMATION PROCESSING DEVICE
- 21: BRAIN INFORMATION INPUT/OUTPUT UNIT
- 22: RECEPTION UNIT
- 23: REPRODUCTION UNIT
- 24: REPRODUCTION CONTROLLER
- 25: TRANSMISSION UNIT
- 26: CONNECTION CONTROLLER
- 30: INFORMATION CONTROLLER
- 31: INFORMATION ACQUISITION UNIT (FIRST INFORMATION ACQUISITION UNIT, AND SECOND INFORMATION ACQUISITION UNIT)
- 32: MULTIPLEXING UNIT
- 33: DETERMINATION UNIT
- 34: INFORMATION CONTROLLER

## Claims

1. An information transmission apparatus comprising:
a first information acquisition unit configured to acquire first brain activity information of a first user;
a second information acquisition unit configured to acquire second brain activity information of a second user;
a determination unit configured to determine whether a difference between the first brain activity information and the second brain activity information is equal to or larger than a preset first determination value; and
an information controller configured to change a capacity of content transmitted and received between the first user and the second user based on a result of the determination by the determination unit.

2. The information transmission apparatus according to claim **1,** wherein
when the determination unit determines that the difference is equal to or larger than the preset first determination value, the information controller is further configured to summarize the content.

3. The information transmission apparatus according to claim 1, wherein
when the determination unit determines that the difference is equal to or larger than the preset first determination value, the information controller is further configured to change a reproduction speed of the content.

4. The information transmission apparatus according to any one of claims 1 to 3, further comprising:
a multiplexing unit configured to multiplex the content, wherein
when the determination unit determines that the difference is equal to or larger than the preset first determination value, the information controller is further configured to transmit the multiplexed content.

5. An information transmission method comprising:
acquiring first brain activity information of a first user;
acquiring second brain activity information of a second user;
determining whether a difference between the first brain activity information and the second brain activity information is equal to or larger than a preset first determination value; and
changing a capacity of content transmitted and received between the first user and the second user based on a result of the determination.

6. A program for causing a computer to execute:
acquiring first brain activity information of a first user;
acquiring second brain activity information of a second user;
determining whether a difference between the first brain activity information and the second brain activity information is equal to or larger than a preset first determination value; and
changing a capacity of content transmitted and received between the first user and the second user based on a result of the determination.
